Europäisches Patentamt

European Patent Office

Office européen des brevets

(19)

(11) Publication number: **0 123 375**
**B1**

(12) **EUROPEAN PATENT SPECIFICATION**

(45) Date of publication of patent specification: **26.10.88**

(51) Int. Cl.⁴: **C 07 K 15/06,** A 61 K 37/04

(21) Application number: **84300717.0**

(22) Date of filing: **06.02.84**

(54) Gamma-globulin preparation for intravenous administration.

(30) Priority: **25.02.83 JP 31460/83**

(43) Date of publication of application:
**31.10.84 Bulletin 84/44**

(45) Publication of the grant of the patent:
**26.10.88 Bulletin 88/43**

(84) Designated Contracting States:
**BE DE FR GB NL SE**

(56) References cited:
**AU-B- 50 599**
**DE-A-1 792 196**
**DE-A-2 751 717**
**GB-A-1 230 127**
**US-A-3 415 804**

**Helwig: "Moderne Arzneimittel" Vol. 5 page 1566 Wissenschaftliche Verlagsgesellschaft Stuttgart 1980**

(73) Proprietor: **THE GREEN CROSS CORPORATION**
**15-1, Imabashi-1-chome Higashi-ku Osaka-shi**
**Osaka 541 (JP)**

(72) Inventor: **Uemura, Yahiro**
**Maison-Hirakata 215 5-18, Mitsuyacho**
**Hirakata-shi (JP)**
Inventor: **Funakoshi, Satoshi**
**3361 San Pasqual Street**
**Pasadena California, 91107 (US)**

(74) Representative: **Harrison, David Christopher**
**et al**
**MEWBURN ELLIS & CO 2/3 Cursitor Street**
**London EC4A 1BQ (GB)**

Courier Press, Leamington Spa, England.

**Description**

This invention relates to an improvement in preserving stability of an intravenously administrable human gamma-globulin dry preparation obtainable by polyethylene gylcol fractionation of the human plasma.

The addition of a stabilizer to aqueous solutions of proteinaceous materials prior to their lophilization is known from, for example, DE-A-1792196. This discloses the use of various saccharides, including glucose, in a stable mixture in which the protein is chelated with a metal.

Polyethylene glycol (hereinafter referred to as PEG) is a substance widely used as a stabilizer or a precipitant for protein and, because of its extremely low toxicity, is used also in the preparation of biological pharmaceuticals.

Thus, DE-A-2751717 describes the extraction of a gamma globulin fraction from Cohn's Fraction II or II + III by a process which includes removing certain materials by treatment with relatively low concentrations of ethanol and polyethylene glycol and thereafter precipitating the gamma globulin using a relatively high concentration of either polyethylene glycol or ethanol. The resultant paste may be redissolved and the solution freeze dried, in which case mannitol may be added to the solution.

US-A-3415804 describes the fractionation of mixtures of proteinaceous substances using polyethylene glycol, including the preparation of pure gamma globulin from blood plasma. The polyethylene glycol may be removed from the gamma globulin by redissolving the precipitate and precipitating the gamma globulin with, e.g. cold ethanol.

Poison et al has purified γ-globulin by adding, to human plasma, PEG to a certain concentration and separating the precipitated protein (US-A-3,415,804). The intravenously administrable γ-globulin preparation is prepared by lyophilizing the product obtained by PEG fractionation mentioned above. But, when the lyophilized preparation is dissolved in distilled water for injection use at the time of administration, it often does not go into solution rapidly.

Some of the present inventors made an extensive study on this point and found that the dissolving velocity was affected by PEG and that when the content of contaminating PEG was below a specified level the dry preparation had an improved water-solubility to dissolve rapidly in water, and proposed a method of producing a plasma protein preparation based on this novel information. In this prior method (unpublished at the priority date of this application) a plasma protein dry preparation comprising human plasma protein is prepared by a PEG fractionation method, but the PEG is removed prior to the drying treatment so as to give a PEG concentration of 0.05% W/V or less when the preparation is dissolved in water to give a protein concentration of about 20% W/V.

However, when the content of PEG in a γ-globulin preparation obtainable by fractionation by use of PEG is decreased, the stability of the γ-globulin against deterioration with time becomes poor accompanied by a decrease in antibody titer and increase in anticomplement activity. We find surprisingly that this tendency can be prevented markedly by addition of a sufficient amount of glucose for stabilising the γ-globulin to an aqueous solution of γ-globulin followed by lyophilization.

Thus, according to this invention, there is provided a method of preparing a dry γ-globulin preparation by fractionating human plasma using polyethylene glycol (PEG), which preparation is suitable for obtaining an intravenously administrable aqueous solution, is substantially free of remaining polyethylene glycol, and is present in an amount sufficient for stabilizing the γ-globulin, i.e. from about 0.2 to 2.0 parts by weight based on 1 part by weight of the γ-globulin.

Since the method of this invention for obtaining the γ-globulin includes PEG fractionation, the resulting fraction generally contains residual PEG, which is thereafter removed.

Thus, a method in accordance with the present invention for preparing a dry γ-globulin preparation includes the steps of

subjecting human plasma or a medium derived therefrom to PEG fractionation to form an aqueous solution of the γ-globulin,

removing the PEG from the aqueous solution to render it substantially free from PEG,

adding to the aqueous solution glucose as stabilizer, and

lyophilizing the aqueous solution,

the glucose being present in the dry γ-globulin preparation in an amount of 0.2 to 2 parts by weight, based on 1 part by weight of the γ-globulin, and the γ-globulin being present to a final concentration of 5 to 20% W/V of the aqueous solution.

As the method for preparing an intravenously administrable γ-globulin by use of PEG, there may be mentioned, for example, one which comprises adding PEG to concentrations of 4% W/V, 5% W/V and 12% W/V successively. [JP-A-20415/78]. But the present invention is not limited to this method of preparation and can be applied widely for improving intravenously administrable γ-globulin preparations utilizing PEG.

The PEGs referred to in this invention are those which can be utilized for plasma fractionation and generally have an average molecular weight of 3000 to 20000. A γ-globulin preparation may be regarded as "substantially free of PEG" if PEG cannot be detected by a particularly sensitive calorimetry detection method when carried out on a preparation dissolved in an aqueous medium so as to provide an aqueous solution of the γ-globulin in an amount of about 5% W/V in terms of protein. The colorimetric method utilizes the formation of barium-iodine complex resulting from combination of PEG with barium and iodine, which has an absorption in 535 nm band [Microchemical Journal *20*, 190—192 (1975)].

The removal of contaminating PEG in γ-globulin fraction capable of intravenous administration may be carried out according to known methods of protein purification by, for example, fractionation by use of alcohol, salting out, or treatment with a synthetic absorbent such as those of the nonpolar styrene-divinylbenzene type. The alcohol fractionation method is carried out by adding to an aqueous solution containing 1 to 10% W/V of the γ-globulin fraction contaminated with PEG a neutral salt such as sodium chloride and magnesium chloride to a concentration of 0.04 to 0.75 mol, adding 15 to 40% V/V of ethanol thereto, then treating the mixture at PH 5 to 8 at a temperature of 0 to −10°C for 30 minutes to 24 hours, and recovering the precipitate formed. The product is, if desired, subsequently subjected to dialysis to regulate the salt concentration. The salting-out method is carried out by preparing an aqueous solution containing 1 to 10% W/V of plasma protein contaminated with PEG, adding ammonium sulfate thereto to 25 to 70% saturation, treating the resulting mixture at pH 5 to 7 at a temperature of 20 to 0°C for 30 minutes to 24 hours, and recovering the precipitate formed. The product is, if desired, subsequently subjected to dialysis to regulate the salt concentration.

The symbol "% W/V" or "% V/V" means herein a percentage of a solute by weight or by volume per a solution by volume, respectively.

The treatment with a synthetic absorbent is carried out by use of a nonpolar styrene-divinylbenzene copolymer, which has a fine-grained surface and is hydrophobic. Commercially available such adsorbents include HIGH POROUS POLYMER® (made by Mitsubishi Kasei, Inc.), WAZI® (made by Mitsubishi Kasei, Inc.), and AMBERLITE XAD® (made by Rohm and Haas, Inc.). The synthetic adsorbent is used perferably after being washed with 10 to 70% W/V of ethanol, or 0.05 to 1.0 N hydrochloric acid or sodium hydroxide. The adsorption treatment is carried out, batchwise or by a column method, by contacting an aqueous solution of γ-globulin contaminated with PEG as it is with the adsorbent to remove the PEG from the aqueous solution fraction by adsorption on the synthetic adsorbent.

Since the method of alcohol fractionation and that of salting out involve the necessity of removing again the inorganic salt or alcohol used in fractionation by lyophilization or dialysis after the treatment, the treatment with a synthetic adsorbent is preferred.

The γ-globulin solution from which the PEG has been thus removed in sterilely filtered in a conventional manner, then glucose is added to the solution as a stabiliser in an amount sufficient for stabilizing the γ-globulin. The amount to be added is about 0.2 to 2 parts by weight based on 1 part by weight of γ-globulin which is dissolved in a solution of 5 to 20% W/V. The solution is lyophilized to give a dry preparation. The preparation is dispensed so that each unit contains 500 to 10000 mg of γ-globulin according to the package unit. It is stored avoiding a high temperature condition and, when using, dissolved in distilled water for injection use and administered intravenously.

The dosage is generally 500 to 3000 mg in terms of γ-globulin per one time for adults and 250 to 1500 mg for infants.

As a safety test, an acute toxicity test was carried out. A 10% solution of the preparation was administered in a total amount of 0.5 ml/animal and 1.0 ml/animal to two groups each consisting of 5 mice via the tail vein of the mouse. No abnormality was recognized in 7 days' observation.

Since the γ-globulin preparation for intravenous administration of this invention contains no γ-globulin that has been subjected to enzymolysis or chemical modification and is substantially of naturally occurring form, it has the advantages of long half-life in blood, no trouble due to antigenicity, and moreover an excellent solubility and high stability against deterioration with time. Thus, it is highly advantageous as a γ-globulin preparation for intravenous administration.

This invention will be illustrated in detail below with reference to an Example and Test Example, but it is not limited thereto.

In the Examples, the measles antibody titer was determined by the hemagglutination inhibition test and expressed in terms of the international unit (IU/100 mg). The anticomplement activity was determined according to Kabatt and Meyer [Experimental Immunochemistry, 225 (1961)] and Nishioka and Okada [Men'eki no Seikagaku (Biochemistry of Immunity), 103 (1971) (published by Kyoritus Shuppan, Inc., Japan)]. Namely, 100 units of a complement was mised with a sample to be tested, and the units remaining after decreasing was measured. The anticomplement activity was expressed in terms of the decrease in units.

Example

A γ-globulin for intravenous administration (measles antibody titer: 9.2 IU/100 mg, anticomplement activity: 15) fractionated by use of Polyethylene Glycol #4000 was dissolved in a 0.02 M acetate buffer solution, ph 7.0, containing 0.5% of sodium chloride to a protein concentration of 5% W/V. This solution was contaminated with 0.2% W/V of Polyethylene Glycol #4000 used in fractionation. Six thousand ml of the γ-globulin solution was passed through a column of 100 ml volume packed with HIGH POROUS POLYMER-HP20 and the fractions containing γ-globulin were pooled. No Polyethylene Glycol #4000 was detected in these γ-globulin fractions according to the colorimetric method already mentioned. Glucose was added to the pooled γ-globulin solution to give a concentration of 2% W/V, and the mixed solution was lyophilized. When 1000 mg of the lyophilized product was dissolved in 15 ml of distilled water for injection use, it dissolved readily. After being stored at 30°C for 10 months, the dried preparation showed neither a

## 0 123 375

decrease in antibody titer nor an increase in anti-complement activity.

Experimental Example

To 100 ml of 5% W/V solution of the pooled γ-globulin obtained in Example 1 was added glucose to give glucose concentrations of 0.1, 0.5, 1, 3, and 10% W/V respectively, and the solution was lyophilized to obtain a dried preparation. The γ-globulin solution showed a measles antibody titer of 9 IU/100 mg and an anticomplement activity of 15. No polyethylene glycol was detected by the colorimetry.

The dry preparation was stored aseptically at 30°C for 5 days and then further for 5 months aseptically. The antibody titer and anticomplement activity was measured after each period. The results were as shown in the following table. Statistically significant effects were observed in each of the tests.

| Glucose added (% W/V) | 30°C, 5 days Storage | | 5 months Storage | |
|---|---|---|---|---|
| | Measles antibody titer | Anti-complement activity | Measles antibody titer | Anti-complement activity |
| 0.1 | 7.5 | 20 | 7.4 | 32 |
| 0.5 | 8.8 | 22 | 8.1 | 24 |
| 1 | 9.1 | 17 | 9.0 | 17 |
| 3 | 9.0 | 17 | 9.0 | 16 |
| 10 | 8.9 | 16 | 9.0 | 17 |
| 0 | 6 | 22 | 8.1 | 35 |

### Claims

1. A method of preparing a dry γ-globulin preparation from human plasma or a medium derived therefrom, which method includes the steps of subjecting the said plasma or medium derived therefrom to polyethylene glycol fractionation to provide an injectable aqueous solution of the γ-globulin and lyophilizing the aqueous solution, characterised in that the method includes the additional steps of removing the polyethylene glycol from the said aqueous solution to render the said aqueous solution substantially free from polyethylene glycol, and adding to the aqueous solution glucose as stabilizer, the glucose being present in an amount of from 0.2 to 2 parts by weight, based on 1 part by weight of the γ-globulin and the γ-globulin being present to a final concentration of 5 to 20% W/V of the aqueous solution.

2. A method according to claim 1, wherein the step of removing the polyethylene glycol is carried out by treating the said aqueous solution with a nonpolar synthetic absorbent.

3. A method according to claim 2, wherein the nonpolar synthetic absorbent is a nonpolar styrenedivinylbenzene type resin.

### Patentansprüche

1. Verfahren zur Herstellung eines trockenen γ-Globulin-Präparats aus menschlichem Plasma oder einem daraus gewonnenen Medium, wobei das Verfahren die folgenden Schritte enthält: Durchführung einer Polyäthylenglykol-Fraktionierung des Plasmas oder des daraus gewonnenen Mediums, um eine injizierbare wäßrige Lösung des γ-Globulins zu erhalten, und Gefriertrocknung der wäßrigen Lösung, dadurch gekennzeichnet, daß das Verfahren die folgenden weiteren Schritte enthält: Entfernung des Polyäthylenglykols aus der wäßrigen Lösung, um die wäßrige Lösung im wesentlichen frei von

4

**0 123 375**

Polyäthylenglykol zu erhalten, und Zugabe von Glukose als Stabilisator zur wäßrigen Lösung, wobei die Glukose in einer Menge von 0,2 bis 2 Gewichtsteilen bezogen auf 1 Gewichtsteil γ-Globulin, und das γ-Globulin in einer Endkonzentration von 5 bis 20 % Gewicht/Volumen der wäßrigen Lösung vorhanden ist.

2. Verfahren nach Anspruch 1, wobei der Schritt der Entfernung des Polyäthylenglykols durch Behandlung der wäßrigen Lösung mit einem nichtpolaren synthetischen Absorbens durch-geführt wird.

3. Verfahren nach Anspruch 2, wobei das nichtpolare synthetische Absorbens ein nichtpolares Harz vom Styrol-Divinyl-benzol-Typ ist.

## Revendications

1. Procédé de préparation d'une préparation de gamma-globuline sèche à partir de plasma humain ou d'un milieu dérivé de celui-ci, lequel procédé comprend les stades consistant à soumettre ce plasma ou ce milieu dérivé de celui-ci à un fractionnement par le polyéthylène glycol pour réaliser une solution aqueuse injectable de la gamma-globuline et à lyophiliser la solution aqueuse, caractérisé en ce que le procédé comprend les stades supplémentaires d'élimination du polyéthylène glycol de cette solution aqueuse pour rendre cette solution aqueuse pratiquement exempte de polyéthylène glycol, et l'addition à la solution aqueuse de glucose comme stabilisant, le glucose étant présent dans une proportion de 0,2 à 2 parties en poids, pour 1 partie en poids de la gamma-globuline, et la gamma-globuline étant présente à une concentration finale de 5 à 20% P/V de la solution aqueuse.

2. Procédé suivant la revendication 1, dans lequel le stade d'élimination du polyéthylène glycol est effectué en traitant cette solution aqueuse par un absorbant synthétique non polaire.

3. Procédé suivant la revendication 2, dans lequel l'absorbant synthétique non polaire est une résine due type styrène-divinylbenzène non polaire.